# EUROPÄISCHE PATENTANMELDUNG

(11) **EP 0 571 063 A1**
(43) Veröffentlichungstag der Anmeldung: **24.11.1993**
(21) Anmeldenummer: 93250225.5
(22) Anmeldetag: 07.12.1990
(51) Int. Cl.: A61K 9/127, A61K 31/20, A61K 7/00

(54) **Pharmazeutische Präparate**

(30) Priorität: 14.12.1989 DE 3941582
(62) Teilanmeldung aus: 91900173.5
(71) Anmelder: SCHERING AKTIENGESELLSCHAFT, D-13353 Berlin (DE)
(72) Erfinder: Rössling, Georg, Dr., D-13465 Berlin (DE); Sachse, Andreas, D-10589 Berlin (DE); Zaumseil, Rolf-Peter, Dr., D-10777 Berlin (DE); Riedl, Jutta, Dr., D-14057 Berlin (DE)

(57) **Zusammenfassung**

Es werden pharmazeutische Präparate beschrieben, die durch einen Gehalt an in Liposomen-Dispersionen befindlichen α,ω-n-Alkandicarbonsäuren mit 7 bis 13 Kohlenstoffatomen, deren physiologisch verträglichen Salzen oder deren mittels Enzymen der Haut spaltbaren Ester gekennzeichnet sind. Diese Präparate dienen vorzugsweise zur topischen Behandlung von Hauterkrankungen und zur Behandlung der Altershaut.

## Beschreibung

Die Erfindung betrifft pharmazeutische Präparate, welche durch einen Gehalt an in Liposomen-Dispersionen befindlichen α,ω-n-Alkandicarbonsäuren mit 7 bis 13 Kohlenstoffatomen, deren physiologisch verträglichen Salzen und deren mittels Enzymen der Haut spaltbaren Estern gekennzeichnet sind. Diese Präparate sind vorzugsweise in Form einer Lotion, eines Gels oder einer Salbe zubereitet und dienen insbesondere zur topischen Behandlung von Hauterkrankungen.

Es ist bekannt, daß pharmazeutische Präparate die α,ω-n-Alkandicarbonsäuren mit 7 bis 13 Kohlenstoffatomen, deren physiologisch verträgliche Salze oder deren mittels Enzymen der Haut spaltbaren Ester - vorzugsweise in einer Konzentration von 5 bis 30 Gewichtsprozent - enthalten zur Behandlung von Hauterkrankungen wie zum Beispiel der Akne, der hyperpigmentären Dermatosen, der Hauthyperpigmentation, der nichtentzündlichen Dermatosen und zur Behandlung der Altershaut geeignet sind (DE-A 28 17 133, EP-A 0229654, EP-A 0336880, US-A 4,292,326, US-A 4,386,104 und US-A 4,818,768).

Es wurde nun gefunden, daß man überraschenderweise eine ausgezeichnete und gleichmäßige Penetrationsgeschwindigkeit dieser Wirkstoffe durch die Haut erzielt, wenn man dieselben in Liposomen verkapselt. So ist es möglich topisch applizierbare Präparate bereitzustellen, die ihre Wirkung im wesentlichen an den Folikeln entfalten. In Liposomen-Dispersionen befindliche α,ω-n-Alkandicarbonsäuren reichern sich hier an und reduzieren insbesondere die Komodogenese bei gleichzeitig positiver Beeinflussung des entzündlichen Geschehens in der Umgebung. Dadurch daß der Wirkstoff in den Liposomen-Dispersionen befindlich ist, ist es möglich, geringe Mengen an Wirkstoff zu verwenden und trotzdem eine hohe Wirkstoffkonzentration am Wirkort zu erzielen. Erwähnenswert ist auch, daß die liposomal verkapselten Wirkstoffe über einen längeren Zeitraum abgegeben werden (substained release).

Zur Verkapselung der Wirkstoffe in Liposomen können Verfahren verwendet werden, die dem Fachmann an sich wohl bekannt sind. So kann man beispielsweise die α,ω-n-Alkandicarbonsäuren und Liposomen bildende Substanzen in einem organischen Lösungsmittel lösen, die Lösung in eine wässrige Phase eintragen und gegebenenfalls nach Homogenisierung das Lösungsmittel destillativ entfernen. Ublicherweise werden die 1 bis 10 fache Gewichtsmenge Liposomen bildende Substanz pro g Wirkstoff verwendet.

Geeignete Liposomen bildende Substanzen sind insbesondere Phospholipide, wie die Sphingomyeline, die Plasmalogene, die Phosphatidylcholine, die phosphatidylethanolamine, die Phosphatidylserine, die Phosphatidylinosite und die Cardiolipine oder auch Gemische dieser Lipide (Dr. Otto-Albert Neumüller: Römpps Chemie-Lexikon; Franck sche Verlagshandlung, Stuttgart (DE) 2665, 3159, 3920 und 4045) und Gemische dieser Phospholipide mit Cholesterin und/oder Ladungsträgern wie zum Beispiel Stearylamin, Phosphatidsäure, Stearinsäure oder Dicetylphosphat. Hierbei werden vorzugsweise 0, 1 bis 40 Gewichtsprozent und insbesondere 1 bis 20 Gewichtsprozent Phospholipid oder Gemisch bezogen auf die wässrige Phase verwendet. Geeignete Gemische enthalten etwa bis zu 60 Gewichtsprozent Cholesterin und bis zu 15 Gewichtsprozent Ladungsträger. Als Lösungsmittel für die Phospholipide oder Gemische und Wirkstoffe verwendet man vorzugsweise Methanol, Ethanol, Isopropanol, Diethylether. Aceton, Chloroform und Gemische dieser Lösungsmittel.

Da die Lipide oxidationsempfindlich sind, wird das Verfahren zweckmäßigerweise unter einer Inertgasatmosphäre, wie Stickstoff oder Argon durchgeführt und die erhaltenen wässrigen Liposomenlösung durch Zugabe von Antioxidantien, wie Natriumascorbat, Tocopherol oder Natriumhydrogensulfit stabilisiert.

Ferner können die wässrigen Liposomenlösungen noch zusätzliche Hilfsstoffe, wie Bactericide, Konservierungsmittel, Puffersubstanzen oder auch Wirkstoffe enthalten, um Kombinationspräparate herzustellen. Derartige Kombinationspräparate sind beispielsweise solche, die zusätzlich noch Keratolytica wie Salizylsäure oder Harnstoff enthalten.

Die Verkapselung der Wirkstoffe in Liposomen kann unter den gleichen Bedingungen durchgeführt werden, wie die vorbekannten Verfahren dieser Art (Pharmazie in unserer Zeit 11, 1982, 97-108, Pure Appl. Chem., 53, 1981, 2241-2254). Das Verfahren zur Verkapselung der antiandrogenen Wirkstoffe eignet sich sowohl zur Herstellung multilamellarer Liposomen als auch zur Herstellung unilamellarer Liposomen.

Bei dem erfindungsgemäßen Verfahren ist es aber andererseits auch möglich, das Lösungsmittel nicht durch Destillation sondern mittels der bekannten Verfahren der transmembranen Destillation (Chem. Ing. Techn. 56, 1984, 514-521; J. of Membrane Sci., 39, 1988, 25-42; DE-A 33 12 359) und Pervaporation (Swiss Chem. 10, 1988, 45-51; ACS Symposium 281, 1985, 467-478; Chem. Ing. Tech. 60, 1988, 590-603) zu entfernen.

Die so dargestellten wirkstoffhaltigen Liposomensuspensionen können bei Bedarf mittels Wasser verdünnt und/oder mit Verdickungsmitteln, wie Hydroxyethylzellulose, Methylzellulose, Aerosil® (Hersteller Degussa AG, DE-6000 Frankfurt) Carbopol® (B.F. Goodrich Chem., USA 44131 Cleveland/Ohio) etc. versetzt werden um so streichfähige Gele herzustellen.

Andererseits ist es aber auch beispielsweise möglich, die Suspensionen mittels Gefriertrocknung zur Trockne einzuengen und den erhaltenen Rückstand in eine Salbengrundlage oder eine Creme einzuarbeiten.

Die optimale Wirkstoffkonzentration in den fertigen pharmazeutischen Präparaten ist von der Art des Wirkstoffs und der galenischen Zubereitung abhängig und muß im Einzelfalle mittels der üblichen Vorversuche ermittelt werden. In der Regel wird es ausreichend sein, wenn man pharmazeutische Präparate anwendet, die 1 mg bis 250 mg Wirkstoff pro Gramm des Präparats verwendet.

Die nachfolgenden Ausführungsbeispiele dienen zur näheren Erläuterung der Erfindung.

### Beispiel 1 (Filmmethode mit anschließender Hochdruck-Homogenisation.)

2,0 g PC S 100 (Hersteller Lipoid KG, DE-Ludwigshafen), 0,2 g Cholesterin und 1,0 g Azelainsäure werden in 100 ml 95 %igem Ethanol gelöst. Dann wird die Lösung in einem 500 ml Rundkolben am Rotationsverdampfer zur Trockne eingeengt, wobei sich ein Lipidfilm an der Glaswand ausbildet. Dieser Lipidfilm wird mit 10 ml 0,015 M wässrigen Zitrat-Puffer (pH 7,4) abgelöst und mit 3 ml 0,1 mol Natronlauge auf pH 6,5 eingestellt. Anschließend wird die erhaltene Liposomensuspension mit einem Hochdruckhomogenisator (Microfluidizer®, der Firma Microfluid, Corp., USA) bei 400 MPa und 25° C homogenisiert und durch ein Filter von 0,2µm filtriert.

### Beispiel 2 (Herstellung eines liposomalen Lipogels)

Die gemäß Beispiel 1 hergestellten Liposomen-Dispersion werden gefriergetrocknet. Der getrocknete Liposomenkuchen wird mit einer Schlagkreuzmühle zerkleinert und das entstandene Pulver portionsweise mit soviel einer Salbengrundlage verrieben, die aus Vaseline besteht, welche 0,02 % 2,6-Di-tert.-butyl-4-methylphenol (=BHT) als Antioxidans enthält, daß die Wirkstoffkonzentration in der fertigen Salbe bei 100 mg/g liegt.

## Patentansprüche

1. Pharmazeutische Präparate gekennzeichnet durch einen Gehalt an in LiposomenDispersionen befindlichen α,ω-n-Alkandicarbonsäuren mit 7 bis 13 Kohlenstoffatomen, deren physiologisch verträglichen Salzen oder deren mittels Enzymen der Haut spaltbaren Ester.

2. Pharmazeutische Präparate gemäß Patentanspruch 1 in Form einer Lotion, eines Gels oder einer Salbe.

3. Pharmazeutische Präparate gemäß Patentanspruch 1 und 2 zur topischen Behandlung von Hauterkrankungen und zur Behandlung der Altershaut.
